# EUROPEAN PATENT APPLICATION

(11) **EP 3 438 782 A1**
(43) Date of publication of application: **06.02.2019**
(21) Application number: 17199508.7
(22) Date of filing: 01.11.2017
(51) Int. Cl.: G06F 1/16, G02B 27/01

(54) **WEARABLE DEVICE WITH THERMAL IMAGING FUNCTION**

(30) Priority: 01.08.2017 TW 106211322 U
(71) Applicant: Leapsy International Ltd., 235 New Taipei City (TW); Wang, Po-Wen, 235 New Taipei City (TW)
(72) Inventor: WANG, PO-WEN, 235 New Taipei City (TW)
(74) Representative: Lang, Christian

(57) **Abstract**

A wearable device of the invention includes a first display device, a second display device near the first device, a camera module catching a visible image in a view angle and transmitting the visible image to the first display device, wherein the visible image is displayed on the first display device, a thermal graphic camera catching a thermal image in the view angle and transmitting the thermal image to the second display device, wherein the thermal image is displayed on the second display device.

## Description

### BACKGROUND OF THE INVENTION

### Field of the Invention

The invention relates to a wearable device, and more particularly to a wearable device with thermal imaging function.

### Description of the Related Art

Wearable devices have been very popular recently. In addition to smart watches and smart bracelets, the head-mounted computer are also very popular ones. The head-mounted computer can execute programs for virtual reality and augmented reality. The wearable devices may have many applications.

### BRIEF SUMMARY OF THE INVENTION

An object of the invention is to provide a wearable device with thermal imaging function. The wearable device of the invention includes a camera module for visible light and a thermal graphic camera for thermal graph. The camera module is configured to catch visible image of environment, and the thermal graphic camera is configured to catch thermal image of the environment, thereby detecting temperatures of persons or objects in the environment. The visible image and the thermal image are displayed on the display device of the wearable device to indicate the temperatures of persons or objects in the environment.

The wearable device in accordance with an exemplary embodiment of the invention includes a first display device, a second display device near the first device, a camera module catching a visible image in a view angle and transmitting the visible image to the first display device, wherein the visible image is displayed on the first display device, a thermal graphic camera catching a thermal image in the view angle and transmitting the thermal image to the second display device, wherein the thermal image is displayed on the second display device.

In another exemplary embodiment, the wearable device further includes a control board, wherein the visible image and the thermal image are transmitted to the first display device and the second display device respectively via the control board.

In another exemplary embodiment, the control board is a liquid crystal on silicon driver board.

In another exemplary embodiment, the wearable device further includes a processor coupled to the first display device, the second display device, the camera module and the thermal graphic camera.

In another exemplary embodiment, the wearable device further includes a gesture recognizing module connected to the processor to detect and recognizing a gesture.

In another exemplary embodiment, the processor further includes an input port receiving an image from an external device, and the image is transmitted to the first display device and the second display device and displayed on the first display device and the second display device.

In another exemplary embodiment, the input port is a high definition multimedia interface port.

In another exemplary embodiment, the wearable device further includes a power supply connected to the processor and providing power to the first display device and the second display device via the processor.

In another exemplary embodiment, the wearable device further includes a power control board connected to the processor, and an external power supply is coupled to the processor via the power control board.

In another exemplary embodiment, the wearable device further includes a speaker connected to the processor, wherein the processor transmits audio signals to the speaker.

In another exemplary embodiment, the wearable device further includes a microphone connected to the processor, wherein the microphone receives sound and converts the sound to audio signals, and the audio signals are transmitted to the processor.

In another exemplary embodiment, the wearable device further includes a storage device connected to the processor.

In another exemplary embodiment, the wearable device further includes a wireless transceiver connected to the processor, wherein the wireless transceiver is connected to a management system via a wireless network.

In another exemplary embodiment, the visible image and the thermal image are transmitted to the processor and further transmitted to the management system via the wireless transceiver.

In another exemplary embodiment, the thermal image is displayed by a color set which is selected from a plurality of color sets.

In another exemplary embodiment, the thermal image indicates a temperature in a predetermined temperature range.

A detailed description is given in the following embodiments with reference to the accompanying drawings.

### BRIEF DESCRIPTION OF THE DRAWINGS

The invention can be more fully understood by reading the subsequent detailed description and examples with references made to the accompanying drawings, wherein:

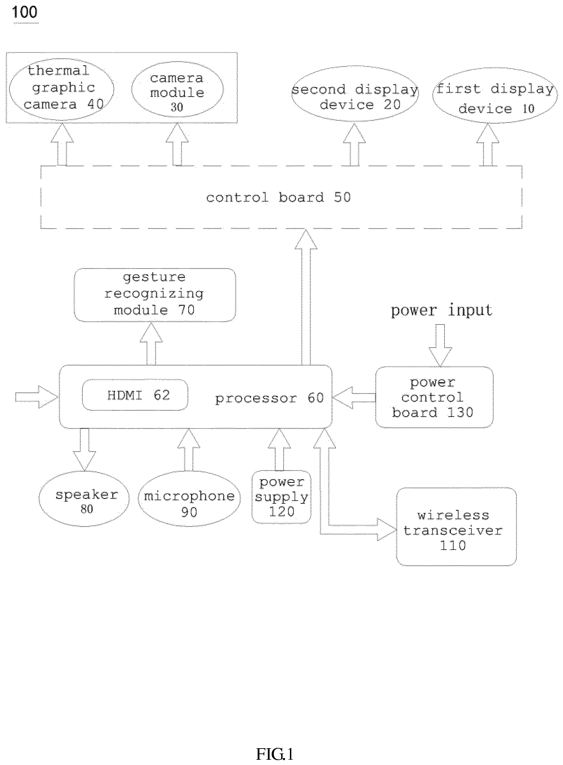

Fig. 1 is a block diagram of an embodiment of a wearable device with thermal imaging function.

### DETAILED DESCRIPTION OF THE INVENTION

The following description is of the best-contemplated mode of carrying out the invention. This description is made for the purpose of illustrating the general principles of the invention and should not be taken in a limiting sense. The scope of the invention is best determined by reference to the appended claims.

Referring to Fig. 1, an embodiment of a wearable device 100 of the invention includes a first display device 10, a second display device 20, a camera module 30 and a thermal graphic camera 40. The second display device 20 is disposed near the first device 10. The camera module 30 catches a visible image in a view angle and transmits the visible image to the first display device 10, and the visible image is displayed on the first display device 10. The thermal graphic camera 40 catches a thermal image in the view angle and transmitting the thermal image to the second display device 20, and the thermal image is displayed on the second display device 20. In this embodiment, the visible image and the thermal image are displayed simultaneously to allow a user to watch the visible image and the thermal image simultaneously so that the user is aware of the temperature of the person or the object that he/she would like to know.

As shown in Fig. 1, the camera module 30 and the thermal graphic camera 40 are coupled to the first display device 10 and the second display device 20 via a control board 50. The visible image caught by the camera module 30 is transmitted to the first display device 10, and the thermal image caught by the thermal graphic camera 40 is transmitted to the second display device 20. In this embodiment, the control board 50 is a liquid crystal on silicon (Lcos) driver board. Therefore, the images caught by the camera module 30 and the thermal graphic camera 40 are transmitted to the first display device 10 and the second display device 20, and a user can watch the images on the first display device 10 and the second display device 20. Since the first display device 10 and the second display device 20 are parallel disposed, the user can watch the visible image and the thermal image at the same time, and compare the visible image with the thermal image to recognize the temperature of persons and objects. The thermal image is displayed by a color set which is selected from a plurality of color sets. For example, red color indicates a higher temperature, and blue color indicates a lower temperature. The colors between the red color and the blue color represent the temperature between a preset high temperature corresponding to the red color and a preset low temperature corresponding to the blue color. The temperature can also be indicated by gray level. That is the white color represents a higher temperature, and the black color represents a lower temperature. In addition, the temperature range indicated by the thermal image can also be preset. For example, the thermal image indicates a highest temperature of 35°C and a lowest temperature of 20°C, and the temperatures between the 35°C and 20°C are represented by different colors. The temperature exceeding 35°C and below 20°C are represented by the color corresponding to 35°C and 20 °C. Therefore, the thermal image has a color variation in a desired range. For example, the temperature range is set to be 31°C to 37°C when human body temperature is detected. The persons who have body temperature exceeding 37°C can be recognized by the thermal image.

As shown in Fig. 1, the wearable device 100 further includes a processor 60. The processor 60 is connected to the control board 50 and coupled to the first display device 10, the second display device 20, the camera module 30 and the thermal graphic camera 40 via the control board 50. The processor 60 includes an input port 62 receiving an image from an external device, and the image is transmitted to the first display device 10 and the second display device 20 and displayed on the first display device 10 and the second display device 20. In this embodiment, the input port 62 is a high definition multimedia interface port (HDMI port). The wearable device 100 further includes a gesture recognizing module 70 connected to the processor 60 and configured to detect and recognizing a gesture. When the gesture recognizing module 70 detects a gesture, an appropriate algorithm is used to recognize the gesture and output a control signal accordingly. For example, a user controls a virtual reality or an augmented reality program such as a game executed in the wearable device 100 by his/her gesture.

In addition, the wearable device 100 further includes a speaker 80 connected to the processor 60, and the processor 60 transmits audio signals to the speaker 80. The wearable device 100 further includes a microphone 90 connected to the processor 60, and the microphone 90 receives sound and converts the sound into audio signals, and the audio signals are transmitted to the processor 60. The user can hear sound from the speaker 80 and also can input sound to the microphone 90. The wearable device 100 further includes a wireless transceiver 110 connected to the processor 60, wherein the wireless transceiver 110 is connected to a management system via a wireless network. The visible image caught by the camera module 30 and the thermal image caught by the thermal graphic camera 40 are transmitted to the processor 60 and further transmitted to the management system via the wireless transceiver 110 so that managing persons may watch the visible image and the thermal image synchronously via the management system and do some proper management. For example, the managing person can monitor the body temperature of persons in the environment. The user can also communicates with the managing person via the wireless transceiver 110.

In addition, the wearable device 100 further includes a power supply 120 connected to the processor 60 and providing power to the first display device 10 and the second display device 20 via the processor 60. In this embodiment, the power supply 120 is a battery. In another embodiment, the wearable device 100 further includes a power control board 130 connected to the processor 60, and an external power supply is coupled to the processor 60 via the power control board 130. The external power supply may provide power for the wearable device 100 or charge the power supply 120.

In addition, the wearable device 100 further includes a storage device connected to the processor 60 such as a memory card in which program codes or data are stored and accessed by the processor 60.

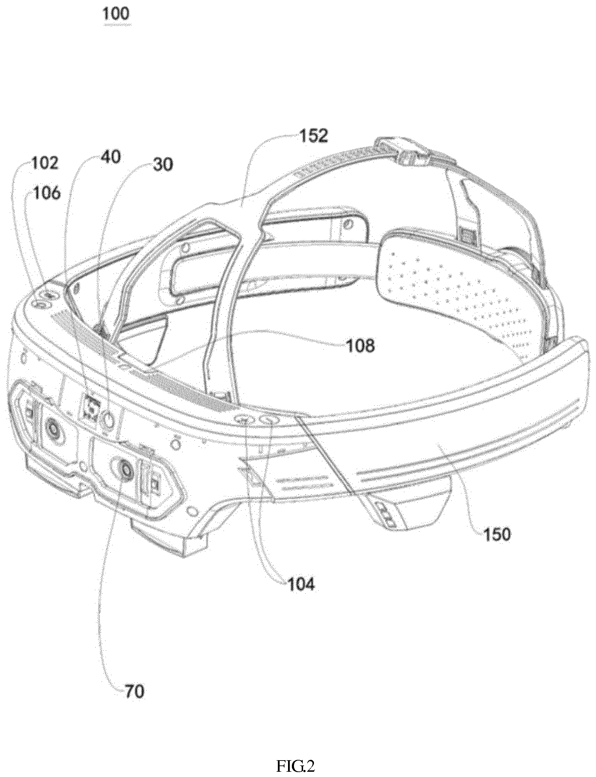

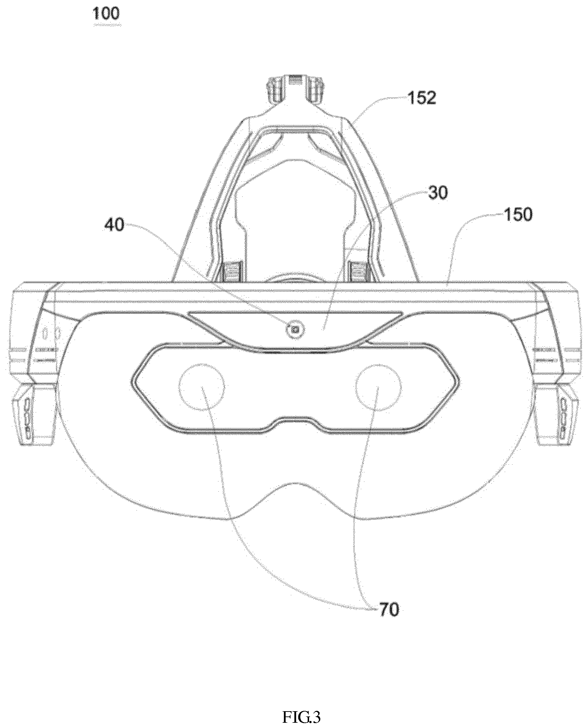

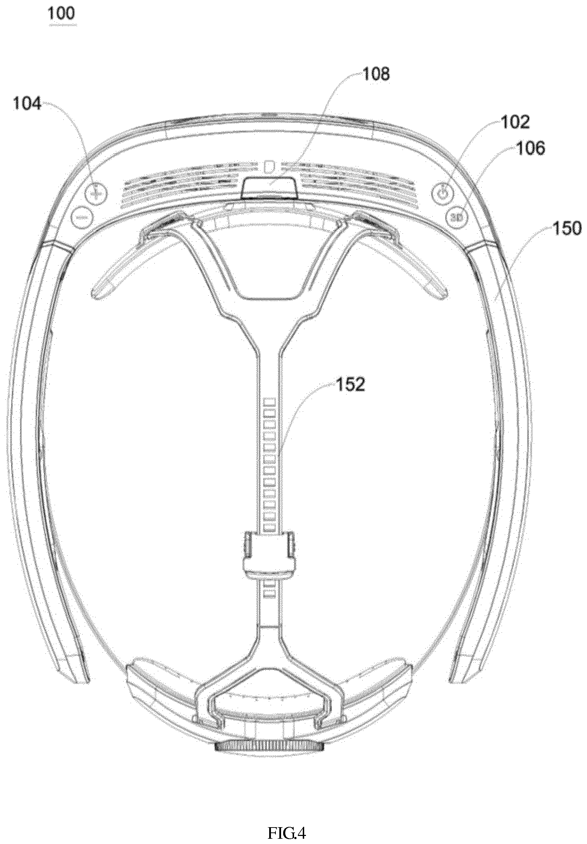

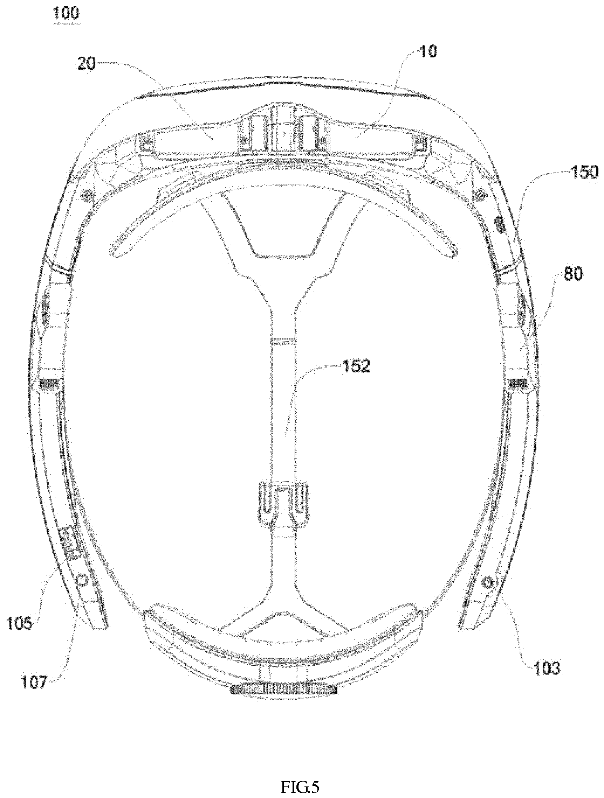

Referring to Figs. 2, 3, 4 and 5, the wearable device 100 further includes a main body 150. The main body 150 is arced and includes a head mounting portion 152. The head mounting portion 152 is mounted to a head of a user. The camera module 30 and the thermal graphic camera 40 are disposed on a front of the main body 150. The gesture recognizing module 70 is also disposed on the front of the main body 150 and under the camera module 30 and the thermal graphic camera 40. The wearable device 100 further includes a power button 102, a sound volume adjusting button 104 and a two dimension/three dimension transforming button 106 disposed on an upper surface of the main body 150. The power button 102 is used to turn on or turn off the wearable device 100, the sound volume adjusting button 104 is used to control the volume of the speaker 80 or earphone, and the two dimension/three dimension transforming button 106 is used to transform the game or other application programs between two dimensional mode and three dimensional mode. A slot 108 is formed on the upper surface of the main body 150 to receive a memory card. In addition, a subscriber identity module (SIM) card is disposed in the main body 150 so that the wearable device 100 may serve as a mobile phone. A earphone hole 103, a USB hole 105 and a hole 107 for the external power supply are formed on a lower surface of the main body 150.

The wearable device 100 of the invention includes the camera module 30 and the thermal graphic camera 40. The camera module 30 catches the visible image and the thermal graphic camera 40 catches the thermal image. The visible image and the thermal image are displayed on the first and second display devices to indicate the temperature of the persons and objects in the environment.

While the invention has been described by way of example and in terms of preferred embodiment, it is to be understood that the invention is not limited thereto. To the contrary, it is intended to cover various modifications and similar arrangements (as would be apparent to those skilled in the art). Therefore, the scope of the appended claims should be accorded the broadest interpretation so as to encompass all such modifications and similar arrangements.

## Claims

1. A wearable device, comprising:
a main body comprising a head mounting portion;
a first display device disposed on the main body;
a second display device disposed on the main body and near the first device;
a camera module configured to catch a visible image in a view angle and transmit the visible image to the first display device, wherein the visible image is displayed on the first display device;
a thermal graphic camera configured to catch a thermal image in the view angle and transmit the thermal image to the second display device, wherein the thermal image is displayed on the second display device; and
a gesture recognizing module connected to the processor and configured to detect and recognizing a gesture.

2. The wearable device as claimed in claim 1, further comprising a control board, wherein the visible image and the thermal image are transmitted to the first display device and the second display device respectively via the control board.

3. The wearable device as claimed in claim 1, further comprising a processor coupled to the first display device, the second display device, the camera module and the thermal graphic camera.

4. The wearable device as claimed in claim 3, wherein the processor further comprises an input port configured to receive an image from an external device, and the image is transmitted to the first display device and the second display device and displayed on the first display device and the second display device.

5. The wearable device as claimed in claim 3, further comprising a power supply connected to the processor and providing power to the first display device and the second display device via the processor.

6. The wearable device as claimed in claim 3, further comprising a power control board connected to the processor, and an external power supply is coupled to the processor via the power control board.

7. The wearable device as claimed in claim 3, further comprising a speaker connected to the processor and a sound volume adjusting button, wherein the processor transmits audio signals to the speaker, and the sound volume adjusting button is configured to adjust sound volume of the speaker.

8. The wearable device as claimed in claim 3, further comprising a microphone connected to the processor, wherein the microphone receives sound and converts the sound to audio signals, and the audio signals are transmitted to the processor.

9. The wearable device as claimed in claim 3, further comprising a storage device connected to the processor, the storage device is a memory card, and the main body further comprises a slot configured to receive the memory card.

10. The wearable device as claimed in claim 3, further comprising a wireless transceiver connected to the processor, wherein the wireless transceiver is connected to a management system via a wireless network, and the visible image and the thermal image are transmitted to the processor and further transmitted to the management system via the wireless transceiver.

11. The wearable device as claimed in claim 1, wherein the thermal image is displayed by a color set which is selected from a plurality of color sets.

12. The wearable device as claimed in claim 1, wherein the thermal image indicates a temperature in a predetermined temperature range.

13. The wearable device as claimed in claim 1, further comprising a power button disposed on the main body.

14. The wearable device as claimed in claim 1, further comprising a two dimension/three dimension transforming button disposed on the main body.

15. The wearable device as claimed in claim 1, further comprising a subscriber identity module disposed in the main body.

## Amended claims

### Amended claims in accordance with Rule 137(2) EPC.

1. A wearable device (100), comprising:
a main body (150) comprising a head mounting portion (152);
a first display device (10) disposed on the main body (150);
a second display device (20) disposed on the main body (150) and near the first device (10);
a camera module (30) configured to catch a visible image in a view angle and transmit the visible image to the first display device (10), wherein the visible image is displayed on the first display device (10); and
a thermal graphic camera (40) configured to catch a thermal image in the view angle and transmit the thermal image to the second display device (20), wherein the thermal image is displayed on the second display device (20);
**characterized in that**
the wearable device (100) further comprises a gesture recognizing module (70) configured to detect and recognizing a gesture, wherein said gesture recognizing module (70) is disposed on the main body (150) and on the back of the first display device (10) and the back of the second display device (20).

2. The wearable device (100) as claimed in claim 1, further comprising a control board (50), wherein the visible image and the thermal image are transmitted to the first display device (10) and the second display device (20) respectively via the control board (50).

3. The wearable device (100) as claimed in claim 1, further comprising a processor (60) coupled to the first display device (10), the second display device (20), the camera module (30) and the thermal graphic camera (40), the processor (60) being further connected to the gesture recognizing module (70).

4. The wearable device (100) as claimed in claim 3, wherein the processor (60) further comprises an input port (62) configured to receive an image from an external device, and the image is transmitted to the first display device (10) and the second display device (20) and displayed on the first display device (10) and the second display device (20).

5. The wearable device (100) as claimed in claim 3, further comprising a power supply (120) connected to the processor (60) and providing power to the first display device (10) and the second display device (20) via the processor (60).

6. The wearable device (100) as claimed in claim 3, further comprising a power control board (130) connected to the processor (60), and an external power supply is coupled to the processor (60) via the power control board (130).

7. The wearable device (100) as claimed in claim 3, further comprising a speaker (80) connected to the processor (60) and a sound volume adjusting button (104), wherein the processor (60) transmits audio signals to the speaker (80), and the sound volume adjusting button (104) is configured to adjust sound volume of the speaker (80).

8. The wearable device (100) as claimed in claim 3, further comprising a microphone (90) connected to the processor (60), wherein the microphone (90) receives sound and converts the sound to audio signals, and the audio signals are transmitted to the processor (60).

9. The wearable device (100) as claimed in claim 3, further comprising a storage device connected to the processor (60), the storage device is a memory card, and the main body (150) further comprises a slot (108) configured to receive the memory card.

10. The wearable device (100) as claimed in claim 3, further comprising a wireless transceiver (110) connected to the processor (60), wherein the wireless transceiver (110) is connected to a management system via a wireless network, and the visible image and the thermal image are transmitted to the processor (60) and further transmitted to the management system via the wireless transceiver (110).

11. The wearable device (100) as claimed in claim 1, wherein the thermal image is displayed by a color set which is selected from a plurality of color sets.

12. The wearable device (100) as claimed in claim 1, wherein the thermal image indicates a temperature in a predetermined temperature range.

13. The wearable device (100) as claimed in claim 1, further comprising a power button (102) disposed on the main body (150).

14. The wearable device (100) as claimed in claim 1, further comprising a two dimension/three dimension transforming button (106) disposed on the main body (150).

15. The wearable device (100) as claimed in claim 1, further comprising a subscriber identity module disposed in the main body (150).
